# EUROPEAN PATENT SPECIFICATION

(11) **EP 4 213 745 B1**
(45) Date of publication and mention of the grant of the patent: **25.02.2026**
(21) Application number: 21870040.9
(22) Date of filing: 14.09.2021
(51) Int. Cl.: A61B 17/22, A61B 17/221, A61F 2/01, A61F 2/06, A61B 17/00, A61M 39/02, A61M 25/06

(54) **DEVICES AND SYSTEMS FOR THROMBUS REMOVAL**
VORRICHTUNGEN UND SYSTEME ZUR THROMBUSENTFERNUNG
DISPOSITIFS ET SYSTÈMES POUR L'ÉLIMINATION DE THROMBUS

(30) Priority: 15.09.2020 US 202063078706 P
(43) Date of publication of application: 26.07.2023
(73) Proprietor: Becton, Dickinson and Company, Franklin Lakes, NJ 07417 (US)
(72) Inventor: MCLAUGHLIN, William Robert, Bountiful, Utah 84010 (US); SOWARDS, Steffan, Salt Lake City, Utah 84124 (US); ANDERSON, Devan, Taylorsville, Utah 84123 (US); MISENER, Anthony, Bountiful, Utah (US)
(74) Representative: dompatent
(86) International application number: PCT/US2021/050156
(87) International publication number: WO 2022/060684

(56) References cited:
- WO-A1-2019/157311
- WO-A1-97/27808
- US-A- 5 779 716
- US-A1- 2003 208 199
- US-A1- 2005 119 668
- US-A1- 2005 171 566
- US-A1- 2006 116 716
- US-A1- 2011 040 314
- US-A1- 2017 258 481
- US-A1- 2018 085 129

## Description

### CROSS-REFERENCE TO RELATED APPLICATION

The present application claims priority to United States Provisional Application No. 63/078,706, entitled "Method of Thrombus Removal and Related Devices and Systems", filed September 15, Z 2020.

### BACKGROUND

A catheter is commonly used to infuse fluids into vasculature of a patient. For example, the catheter may be used for infusing normal saline solution, various medicaments, or total parenteral nutrition. The catheter may also be used for withdrawing blood from the patient.

The catheter may include an over-the-needle peripheral intravenous ("IV") catheter. In this case, the catheter may be mounted over an introducer needle having a sharp distal end. The catheter and the introducer needle may be assembled so that the distal end of the introducer needle extends beyond the distal end of the catheter with the bevel of the needle facing up away from skin of the patient. The catheter and the introducer needle are generally inserted at a shallow angle through the skin into vasculature of the patient.

In order to verify proper placement of the introducer needle and/or the catheter in the blood vessel, a clinician generally confirms that there is "flashback" of blood in a flashback chamber of the catheter assembly. Once placement of the needle has been confirmed, the clinician may remove the needle, leaving the catheter in place for future blood withdrawal or fluid infusion.

Blood withdrawal using the catheter may be difficult for several reasons, particularly when a dwell time of the catheter within the vasculature is more than one day. When the catheter is left inserted in the patient for a prolonged period of time, the catheter or vein may be more susceptible to narrowing, collapse, kinking, blockage by debris (e.g., a thrombus), and adhering of a tip of the catheter to the vasculature. Due to this, the catheter is often used for acquiring a blood sample at a time of catheter placement, but the catheter is less frequently used for acquiring a blood sample during the catheter dwell period. Therefore, when a blood sample is required, an additional needle stick is often used to provide vein access for blood collection, which may be painful for the patient and result in higher material costs.

In some instances, in order to avoid the additional needle stick, a vascular access instrument may be used to access the vasculature of the patient via the catheter. The vascular access instrument may be inserted through the catheter and into the vasculature to extend a life of the catheter and allow blood withdrawal through the catheter without the additional needle stick.

The subject matter claimed herein is not limited to embodiments that solve any disadvantages or that operate only in environments such as those described above. Rather, this background is only provided to illustrate one example technology area where some implementations described herein may be practiced. The scope of the invention is defined in the claims.

A similar vascular access instrument is known from US 2017/258481. This document discloses an endoscopic stone-extraction device comprising a support filament, an end portion, a sheath, a lumen, wherein the support filament is disposed in the lumen such that the sheath is slide able with respect to the support filament, and a handle comprising an actuator.

### SUMMARY

The vascular access instrument according to the invention is defined in claim 1. Preferred embodiments are defined in the dependent claims.

### FURTHER DISCLOSURE

The present disclosure further relates generally to vascular access devices, systems, and methods. The methods disclosed are not claimed. More particularly, the present disclosure relates to a method of thrombus removal and related devices and systems. In some embodiments, a vascular access instrument may be configured to insert through a vascular access device. In some embodiments, the vascular access instrument may include a wire. In some embodiments, the vascular access instrument may include a proximal end and a distal end opposite the proximal end. In some embodiments, the distal end may include a bent shape, which may facilitate removal of a thrombus.

In some embodiments, the bent shape may include a looped portion. In some embodiments, a center axis extending through the looped portion may be configured to be oriented perpendicular to a longitudinal axis of the vascular access device. In some embodiments, the vascular access instrument may include a middle portion. In some embodiments, the middle portion may be disposed between the proximal end and the distal end and proximate the distal end. In some embodiments, the middle portion may be straight. In some embodiments, a center of the looped portion may be oriented perpendicular to the middle portion. In some embodiments, a distal tip of the distal end may be pointed in a proximal direction.

In some embodiments, the bent shape may include a S-shaped portion proximate a U-shaped portion to form an "umbrella shape." In some embodiments, a mouth of the U-shaped portion may face a proximal direction. In some embodiments, a distal tip of the distal end may be pointed in a proximal direction. In some embodiments, the vascular access instrument may include a middle portion disposed between the proximal end and the distal end and proximate the distal end. In some embodiments, the middle portion is straight. In some embodiments, the S-shaped portion and the U-shaped portion may be generally planar. In some embodiments, a plane of the S-shaped portion and the U-shaped portion may be aligned with the middle portion.

In some embodiments, the bent shape may include a spiral. In some embodiments, a distal tip of the distal end may be pointed in a distal direction. In some embodiments, the spiral may include a first end, a second end, and a middle portion between the first end and the second end, wherein the first end and the second end of the spiral each have an outer diameter less than an outer diameter of the middle portion.

In some embodiments, a vascular access system may include a catheter assembly. In some embodiments, the catheter assembly may include a catheter adapter and a catheter extending distally from the catheter adapter. In some embodiments, the vascular access system may include an instrument advancement device coupled to the catheter assembly. In some embodiments, the instrument advancement device may include the vascular access instrument. In some embodiments, the instrument advancement device may be configured to advance the vascular access instrument from a retracted position within the catheter assembly to an advanced position beyond a distal end of the catheter.

In some embodiments, the vascular access instrument may be delivered through the catheter assembly to vasculature of a patient via an instrument advancement device. In some embodiments, the vascular access instrument may facilitate an increased dwell period of the catheter of the catheter assembly within the vasculature of the patient. In some embodiments, the instrument advancement device may be used to advance the vascular access instrument into the catheter and/or beyond a distal end of the catheter when the catheter is compromised to overcome obstructions such as thrombus, valves, and/or a fibrin sheath in or around the catheter that may otherwise prevent blood draw. In some embodiments, the instrument advancement device may provide needle-free delivery of the vascular access instrument to the vasculature of the patient prior to blood collection, fluid delivery, patient or device monitoring, or other clinical procedures by utilizing an existing catheter dwelling within the vasculature.

In some embodiments, in response to the vascular access instrument advancing from the retracted position to the advanced position, the vascular access instrument may be configured to automatically fold to form a U-shaped distal end, which may facilitate thrombus removal. In some embodiments, in response to the vascular access instrument being in the retracted position, the distal end of the vascular access instrument may be wave shaped.

In some embodiments, the vascular access instrument may include a tube. In some embodiments, an outer diameter of the tube may contact an inner diameter of the catheter. In some embodiments, a distal end of the tube may include a funnel shape.

In some embodiments, a vascular access system, may include a catheter assembly, which may include a catheter adapter and a catheter extending distally from the catheter adapter. In some embodiments, the vascular access system may include an instrument retraction device coupled to the catheter assembly. In some embodiments, the instrument retraction device may include an intraluminal layer. In some embodiments, the intraluminal layer may be configured to retract from a first position to a second position within the catheter assembly. In some embodiments, in response to the intraluminal layer being in the first position, the intraluminal layer curves from an inner portion of the catheter to an outer surface of the catheter around an edge of the catheter forming a distal opening of the catheter. In some embodiments, in response to retracting the intraluminal layer from the first position to the second position, the vascular access instrument straightens.

In some embodiments, the intraluminal layer may include a lining, a catheter, or a coating. In some embodiments, the intraluminal layer disposed at a distal end of the catheter may be formed via dipping the distal end of the catheter in a material. In some embodiments, the intraluminal layer at a distal end of the catheter is constructed of a shape-memory material. In some embodiments, the intraluminal layer may be constructed of wire, fiber, or mesh. In some embodiments, the intraluminal layer may include a coil. In some embodiments, the intraluminal layer may include multiple concentric materials.

It is to be understood that both the foregoing general description and the following detailed description are examples and explanatory and are not restrictive of the present invention, as claimed. It should be understood that the various embodiments are not limited to the arrangements and vascular access instrumentality shown in the drawings. Also, the drawings are not necessarily to scale. It should also be understood that the embodiments may be combined. For example, one or more features of a particular vascular access instrument may be combined with one or more features of another particular vascular access instrument. It should also be understood that other embodiments may be utilized and that structural changes, unless so claimed, may be made without departing from the scope of the various embodiments of the present disclosure. The following detailed description is, therefore, not to be taken in a limiting sense.

### BRIEF DESCRIPTION OF THE DRAWINGS

Example embodiments will be described and explained with additional specificity and detail through the use of the accompanying drawings in which:
Figure 1A is an upper perspective view of an example vascular access system, illustrating an example vascular access instrument in an example retracted position, according to some embodiments;
Figure 1B is an upper perspective view of the vascular access system, illustrating the vascular access instrument in an example advanced position, according to some embodiments;
Figure 2A is a side view of a portion of the vascular access system according to the invention, illustrating the vascular access instrument in the advanced position;
Figure 2B is a side view of a portion of the vascular access system, illustrating another example vascular access instrument in the advanced position, according to some embodiments;
Figure 2C is a side view of a portion of the vascular access system, illustrating another example vascular access instrument in the advanced position, according to some embodiments;
Figure 2D is a distal end view of the vascular access instrument of Figure 2C, illustrating the vascular access instrument in the advanced position, according to some embodiments;
Figure 3A is a side view of a portion of the vascular access system, illustrating another example vascular access instrument moving from the proximal position to the advanced position, according to some embodiments;
Figure 3B is a side view of a portion of the vascular access system, illustrating the vascular access instrument of Figure 3A advanced further in a distal direction than Figure 3A, according to some embodiments;
Figure 3C is a side view of the portion of the vascular access system, illustrating the vascular access instrument of Figure 3A in the advanced position, according to some embodiments;
Figure 4 is an upper perspective view of another example vascular access instrument, according to some embodiments;
Figure 5 is an upper perspective view of another example vascular instrument, according to some embodiments;
Figure 6A is a cross-sectional view of an example intraluminal layer, according to some embodiments;
Figure 6B is a cross-sectional view of the intraluminal layer moving from a first position to a second position, according to some embodiments; and
Figure 6C is a cross-sectional view of the intraluminal layer moved proximally from Figure 6B, according to some embodiments.

### DESCRIPTION OF EMBODIMENTS

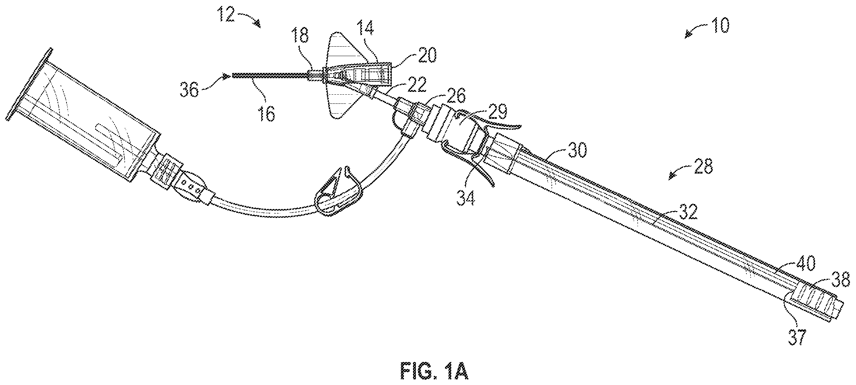

Referring now to Figures 1A-1B, a vascular access system 10 is illustrated, according to some embodiments. In some embodiments, the vascular access system 10 may include a catheter assembly 12, which may include a catheter adapter 14 and a catheter 16. In some embodiments, the catheter 16 may include a peripheral intravenous catheter, a peripherally-inserted central catheter, or a midline catheter. In some embodiments, the catheter adapter 14 may include a distal end 18, a proximal end 20, and a lumen extending through the distal end 18 and the proximal end 20. In some embodiments, the catheter 16 may extend distally from the distal end 18 of the catheter adapter 14.

In some embodiments, the catheter adapter 14 may be integrated with an extension tube 22, which may extend from a side port 24 of the catheter adapter 14. In some embodiments, an adapter 26, such as a Y-adapter or a T-adapter, for example, may be coupled to a proximal end of the extension tube 22.

In some embodiments, an instrument advancement device 28 may be coupled to the catheter assembly 12 in various ways. As an example, the instrument advancement device 28 may be coupled to a port of the adapter 26. As another example, the instrument advancement device 28 may be coupled to a needleless connector 29 disposed between the port of the adapter 26 and the instrument advancement device 28. As another example, the instrument advancement device 28 may be coupled to the proximal end 20 of the catheter adapter 14. In some embodiments, another extension tube and/or a blood collection device adapter may be coupled to another port of the adapter 26. In some embodiments, the blood collection device adapter may receive a blood collection device, such as, for example, a syringe or a blood collection tube.

In some embodiments, the instrument advancement device 28 may include a housing 30 configured to couple to the catheter assembly 12. In some embodiments, the instrument advancement device 28 may include a vascular access instrument 32. In some embodiments, the instrument advancement device 28 may include any suitable delivery device. Some examples of instrument advancement devices that may be used with the vascular access instrument 32 are described further in in U.S. Patent Publication No. 2019/0021640, U.S. Patent Publication No. 2019/0321595, U.S. Patent Publication No. 2019/0321590, U.S. Patent Publication No. 2020/0016374, U.S. Patent Publication No. 2020/0170559, U.S. Patent Application No. 62/794,437, filed January 18, 2019, entitled "CATHETER DELIVERY DEVICE AND RELATED SYSTEMS AND METHODS," and U.S. Patent Application No. 62/830,286, filed April 5, 2019, entitled "VASCULAR ACCESS INSTRUMENT HAVING A FLUID PERMEABLE STRUCTURE AND RELATED DEVICES AND "METHODS".

In some embodiments, the instrument advancement device 28 may be configured to introduce the vascular access instrument 32 into the catheter assembly 12. In some embodiments, in response to the vascular access instrument 32 being introduced into the catheter assembly 12, the vascular access instrument 32 may access a fluid path of the catheter assembly 12 and/or the vascular access instrument 32 may extend through the catheter assembly 12 to access the vasculature of the patient.

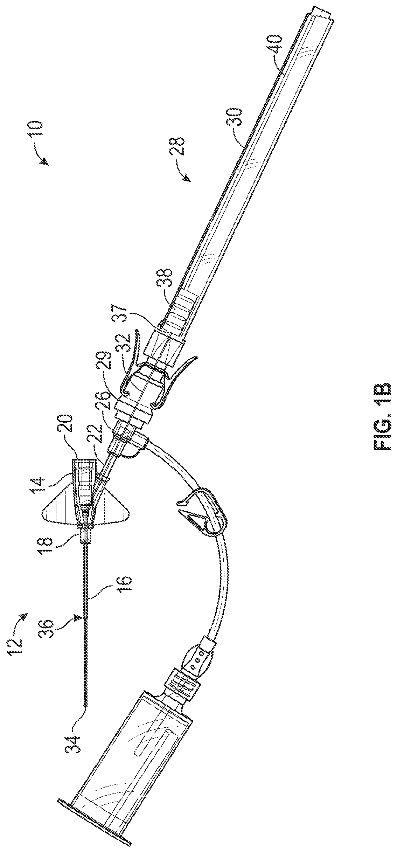

In some embodiments, the instrument advancement device 28 may be configured to advance the vascular access instrument 32 between a retracted position, illustrated, for example, in Figure 1A, to an advanced position, illustrated, for example, in Figure 1B. In some embodiments, a distal end 34 of the vascular access instrument 32 may be disposed distal to a distal end 36 of the catheter 16 in response to the vascular access instrument 32 being in the advanced position. In some embodiments, in response to the vascular access instrument 32 being in the retracted position, the distal end 34 of the vascular access instrument 32 may be disposed within the housing 30. In some embodiments, a proximal end 37 of the vascular access instrument 32 may be coupled to an advancement tab 38, which may be gripped and moved along a slot 40 by a user to move the vascular access instrument 32 between the retracted position and the advanced position. In some embodiments, the advancement tab 38 may extend through the slot 40, and a portion of the advancement tab 38 coupled to the proximal end of the vascular access instrument 32 may be within the housing 30.

In some embodiments, the catheter 16 may be constructed of fluorinated ethylene propylene, TEFLON^{™}, silicon, thermoplastic elastomer, thermoplastic polyurethane, a fluorinated polymer, a hydrophilic material, a hydrophobic material, an anti-fouling material, or another suitable material. In some embodiments, the catheter 16 may include an anti-thrombogenic coating. In some embodiments, all or a portion of the vascular access instrument 32 may be constructed of metal or another suitable material.

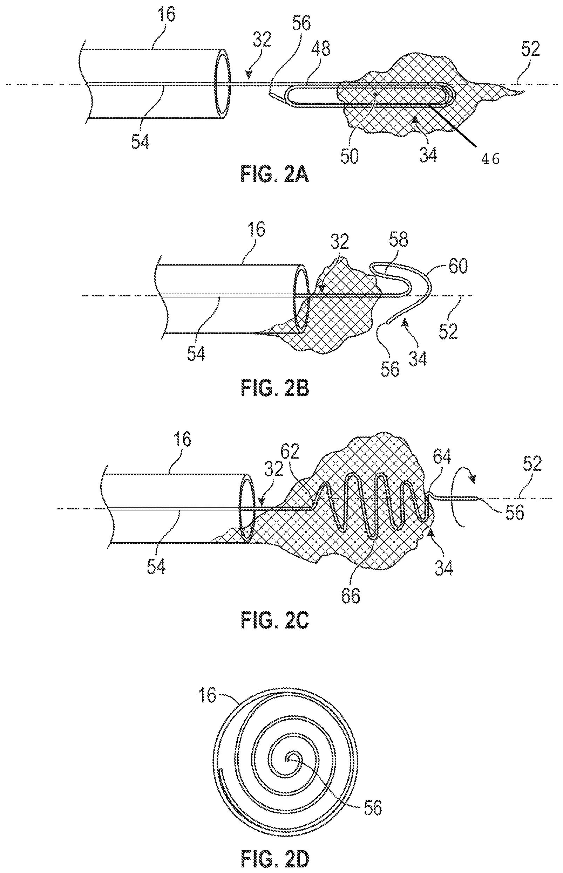

Referring now to Figures 2A-2D, in some embodiments, the vascular access instrument 32 may include a wire or another suitable instrument. In some embodiments, the vascular access instrument 32 may include the proximal end 37 (see Figures 1A-1B, for example) and the distal end 34 opposite the proximal end 37. In some embodiments, the distal end 34 may include a bent shape 44, which may facilitate removal of a thrombus disposed at or near the distal end 36 of the catheter 16.

Referring now to Figure 2A, according to the invention, the bent shape 44 includes a looped portion 46. The looped portion 46 includes one or more loops, wherein the looped portion extends parallel with a longitudinal axis of the vascular access device. In some embodiments, the looped portions 46 may include more than one loop. In some embodiments, the looped portion 46 may include less than three loops, which may reduce a thickness of the looped portion and reduce a risk of the looped portion 46 from interfering with a wall of the vasculature. In some embodiments, the looped portion 46 may include more than three loops. In some embodiments, the loops of the looped portion may be measured from a first point of alignment or contact 48 traveling distally along the vascular access instrument 32 from the proximal end 37.

In some embodiments, the loops may be concentric about a center axis 50. In some embodiments, the center axis 50 extending through the looped portion 46 may be configured to be oriented perpendicular to a longitudinal axis 52 of the catheter 16. In some embodiments, the vascular access instrument 32 may include a middle portion 54. In some embodiments, the middle portion 54 may be disposed between the proximal end 37 and the distal end 34 and proximate the distal end 34. In some embodiments, the middle portion 54 may be straight and/or aligned with the longitudinal axis 52. In some embodiments, the center axis 50 of the looped portion 46 may be oriented perpendicular to the middle portion 54.

In some embodiments, a distal tip 56 of the distal end 34 may be pointed in a proximal direction. In some embodiments, the distal tip 56 may include any suitable shape that is at an endmost portion of the vascular access instrument 32. In some embodiments, the distal tip 56 may be blunt or pointed. In some embodiments, the looped portion 46 may be configured to grab or snare an occlusion within the vasculature, such as a thrombus, and/or facilitate removal of the occlusion from the catheter 16 by the user.

Referring now to Figure 2B, in some embodiments, the bent shape may include a S-shaped portion 58 proximate a U-shaped portion 60 to form an "umbrella shape." In some embodiments, a mouth of the U-shaped portion 60 may face a proximal direction. In some embodiments, the looped portion 46 may be configured to grab or snare an occlusion within the vasculature, such as a thrombus, and/or facilitate removal of the occlusion from the catheter 12 by the user. In some embodiments, the S-shaped portion and/or the U-shaped portion may be configured to grab the occlusion and pull move the occlusion distally and/or proximally.

In some embodiments, the distal tip 56 of the distal end 34 may be pointed in the proximal direction, which may decrease a risk of damaging contact of the distal tip 56 with the wall of the vasculature. In some embodiments, the vascular access instrument 32 may include the middle portion 54, which may be disposed between the proximal end 37 and the distal end 34 and proximate the distal end 34. In some embodiments, the middle portion 54 may be straight and/or aligned with the longitudinal axis 52. In some embodiments, the S-shaped portion 58 may be proximate the middle portion 54. In some embodiments, the S-shaped portion 58 and the U-shaped portion 60 may be generally planar. In some embodiments, a plane of the S-shaped portion and the U-shaped portion may be aligned with the middle portion 54.

Referring now to Figures 2C-2D, in some embodiments, the bent shape 44 may include a spiral or coil-shape. In some embodiments, the distal tip 56 of the distal end 34 may be pointed in a distal direction. In some embodiments, the spiral may include a first end 62, a second end 64, and a middle portion 66 between the first end 62 and the second end 64. In some embodiments, the first end 62 and the second end 64 of the spiral each have an outer diameter less than an outer diameter of the middle portion 66. In some embodiments, each of the loops of the spiral may be spaced apart from a next adjacent loop of the loops. In some embodiments, the vascular access instrument 32 may be configured to move between the retracted position and the advanced position. In some embodiments, the vascular access instrument 32 may be configured to rotate up to 360° about the longitudinal axis 52, which may facilitate movement and/or break up of the occlusion.

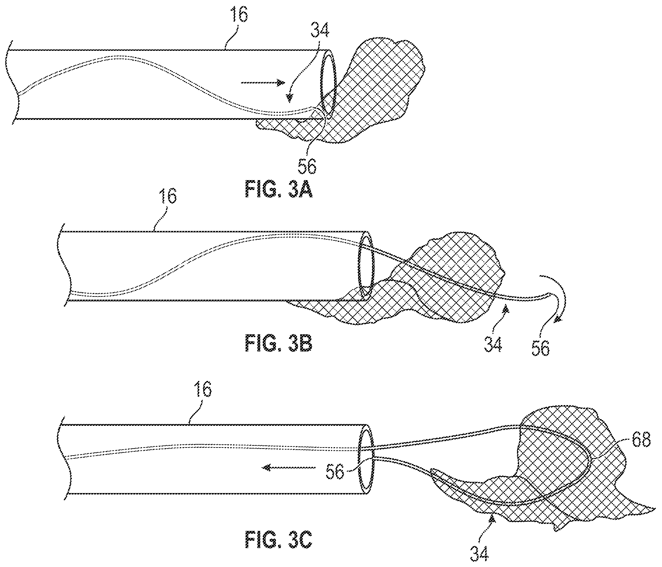

Referring now to Figures 3A-3C, in some embodiments, in response to the vascular access instrument 32 advancing from the retracted position to the advanced position, illustrated, for example, in Figure 3C, the vascular access instrument 32 may be configured to automatically fold to form a U-shape 68, which may facilitate thrombus removal. In some embodiments, in response to the vascular access instrument 32 being in the retracted position, the distal end 34 of the vascular access instrument 32 may be wave shaped or biased in a wave shape by contact with an internal surface of the catheter assembly 12, such as the catheter 16. In some embodiments, in response to formation of the U-shaped distal end, the vascular access instrument 32 may be grab or snare the occlusion within the vasculature, such as a thrombus, and/or facilitate removal of the occlusion from the catheter 16 by the user.

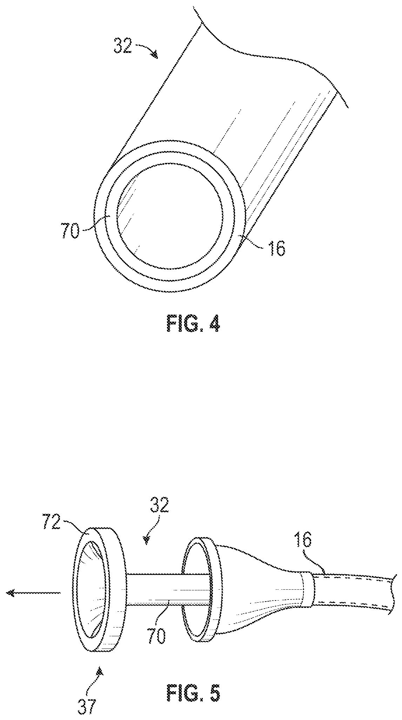

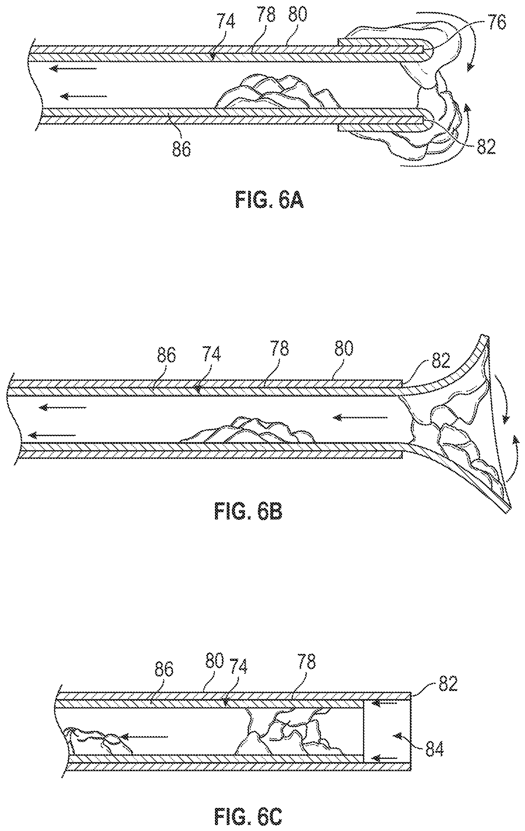

Referring now to Figures 4-5, in some embodiments, the vascular access instrument 32 may include a tube 70. In some embodiments, an outer diameter of the tube 70 may contact an inner diameter of the catheter. In some embodiments, the tube 70 may be cylindrical along all or a portion of a length of the tube 70. In some embodiments, the tube 70 may include or correspond to the intraluminal layer 74 described with respect to Figures 6A-6C. In some embodiments, in response to the vascular access instrument 32 being in the advanced position, a distal end of the tube 70 may be flush or proximal to the distal end of the catheter 16.

In some embodiments, a proximal end of the tube 70 may include a cap 72, which may include a funnel shape and/or enlarged outer diameter. In some embodiments, a fluid pathway may extend through the cap 72. In some embodiments, the cap 72 may be configured to couple to a syringe or intravenous ("IV") line and may be removably coupled to the tube 70. In some embodiments, the cap 72 may include threading, which may facilitate coupling to the syringe of IV line. Figure 5 illustrates the tube 70 in a particular retracted position, according to some embodiments. In some embodiments, in response to movement of the vascular access instrument 32 from the advanced position to the retracted position, the tube 70 and the cap 72 may facilitate breaking up or removal of the occlusion from the catheter 16 by the user. In some embodiments, the cap 72 may be moved proximally, which may pull a remaining portion of the tube 70 proximally. In some embodiments, the tube 70 may correspond to the intraluminal layer 74 of Figures 6A-6C.

Referring now to Figures 6A-6C, the vascular access system 10 may include an instrument retraction device coupled to the catheter assembly 12 (see, for example, Figures 1A-1B). In some embodiments, the instrument retraction device may include or correspond to the instrument advancement device 28 in Figure 1B and may be disposed with the advancement tab 38 in a distal position as in Figure 1B during assembly. In some embodiments, the instrument retraction device may include any suitable instrument retraction device configured to move the vascular access instrument 32 in a proximal direction.

In some embodiments, the vascular access instrument 32 may include an intraluminal layer 74. In some embodiments, the intraluminal layer 74 may be configured to retract from a first position, illustrated, for example, in Figure 6A, to a second or proximal position within the catheter assembly 12. In some embodiments, the intraluminal layer 74 may be disposed in the first position when the instrument retraction device is configured with the advancement tab 38 in the distal position.

In some embodiments, in response to the intraluminal layer 74 being in the first position, the intraluminal layer 74 may include a curved portion 76 that curves from an inner portion 78 of the catheter 16 to an outer surface 80 of the catheter 16 around an edge 82 of the catheter 16 forming a distal opening 84 of the catheter 16. In some embodiments, the curved portion 76 may be coupled and proximate to a tubular portion 86 of the intraluminal layer 74. In some embodiments, in response to retracting the intraluminal layer from the first position to the second position, the curved portion 76 of the intraluminal layer 74 may straighten or become less curved, as illustrated, for example, in Figure 6C.

In some embodiments, the intraluminal layer 74 may include a lining, a catheter, a tube, a coating, or another suitable material configured to move from a curved position to a straight position. In some embodiments, curved portion 76 or the intraluminal layer 74 at a distal end of the catheter 16 may be formed via dipping the distal end of the catheter 16 in a material, which may be flexible. In some embodiments, the intraluminal layer 72 at a distal end of the catheter 16 may be constructed of a shape-memory material. In some embodiments, the intraluminal layer 74 may be constructed of wire, fiber, or mesh. In some embodiments, the intraluminal layer 74 may include a coil. In some embodiments, the intraluminal layer 74 may include multiple concentric materials.

All examples and conditional language recited herein are intended for pedagogical objects to aid the reader in understanding the present disclosure and the concepts contributed by the inventor to furthering the art, and are to be construed as being without limitation to such specifically recited examples and conditions.

## Claims

1. A vascular access instrument (32) configured to insert through a vascular access device (16), the vascular access instrument (32) comprising:
a single wire (32), comprising:
a proximal end (37);
a straight middle portion (54); and
a distal end (34) opposite the proximal end (37), the straight middle portion (54) extending from the proximal end (37) to the distal end (34),
wherein the distal end (34) comprises a bent shape having a looped portion (46),
**characterized in that** the looped portion extends parallel with a longitudinal axis (52) of the vascular access device (16), the looped portion (46) comprising a center axis (50) extending through the looped portion (46) and oriented perpendicular to the longitudinal axis (52) of the vascular access device (16),
wherein the looped portion (46) is configured to grab or snare an occlusion within a vasculature and/or to facilitate removal of the occlusion.

2. The vascular access instrument of claim 1, wherein the middle portion (54) is disposed proximate the distal end (34), wherein a center of the looped portion (66) is oriented perpendicular to the middle portion (54).

3. The vascular access instrument of claim 1, wherein a distal tip (56) of the distal end (34) is pointed in a proximal direction.

4. The vascular access device of claim 1, wherein the looped portion (46) comprises one of a single loop and a plurality of concentric loops.

5. The vascular access device of claim 1, wherein the loops of the looped portion (46) are measured from a first point of alignment or contact (48) traveling distally along the vascular access instrument (32) from the proximal end (37) of the vascular access device and wherein the looped portion (46) is positioned below the longitudinal axis (52) of the vascular access device (16).

## Patentansprüche

1. Gefäßzugangsinstrument (32) , das zum Einführen durch eine Gefäßzugangsvorrichtung (16) ausgebildet ist, wobei das Gefäßzugangsinstrument (32) aufweist:
einen Einzeldraht (32), der aufweist:
ein proximales Ende (37);
einen geraden Mittelabschnitt (54); und
ein distales Ende (34) entgegengesetzt zu dem proximalen Ende (37), wobei sich der gerade Mittelabschnitt (54) vom proximalen Ende (37) zum distalen Ende (34) erstreckt, wobei das distale Ende (34) eine gebogene Form mit einem geschlungenen Abschnitt (46) aufweist,
**dadurch gekennzeichnet, dass** sich der geschlungene Abschnitt parallel zu einer Längsachse (52) der Gefäßzugangsvorrichtung (16) erstreckt, wobei der geschlungene Abschnitt (46) eine Mittelachse (50) aufweist, die sich durch den geschlungenen Abschnitt (46) erstreckt und senkrecht zur Längsachse (52) der Gefäßzugangsvorrichtung (16) ausgerichtet ist,
wobei der geschlungene Abschnitt (46) so ausgebildet ist, dass er eine Okklusion innerhalb eines Gefäßes ergreift oder einschnürt und/oder die Entfernung der Okklusion erleichtert.

2. Gefäßzugangsinstrument nach Anspruch 1, wobei der Mittelabschnitt (54) in der Nähe des distalen Endes (34) angeordnet ist, wobei eine Mitte des geschlungenen Abschnitts (66) senkrecht zum Mittelabschnitt (54) ausgerichtet ist.

3. Gefäßzugangsinstrument nach Anspruch 1, wobei eine distale Spitze (56) des distalen Endes (34) in eine proximale Richtung gerichtet ist.

4. Gefäßzugangsvorrichtung nach Anspruch 1, wobei der geschlungene Abschnitt (46) eine einzelne Schlinge oder eine Vielzahl konzentrischer Schlingen aufweist.

5. Gefäßzugangsvorrichtung nach Anspruch 1, wobei die Schlingen des geschlungenen Abschnitts (46) von einem ersten Ausrichtungs- oder Kontaktpunkt (48) aus gemessen werden, der sich vom proximalen Ende (37) der Gefäßzugangsvorrichtung distal entlang des Gefäßzugangsinstruments (32) erstreckt, und wobei der geschlungene Abschnitt (46) unterhalb der Längsachse (52) der Gefäßzugangsvorrichtung (16) positioniert ist.

## Revendications

1. Instrument d'accès vasculaire (32) configuré pour être inséré à travers un dispositif d'accès vasculaire (16), l'instrument d'accès vasculaire (32) comprenant :
un fil unique (32), comprenant :
une extrémité proximale (37) ;
une partie intermédiaire droite (54) ; et
une extrémité distale (34) opposée à l'extrémité proximale (37), la partie intermédiaire droite (54) s'étendant de l'extrémité proximale (37) à l'extrémité distale (34),
dans lequel l'extrémité distale (34) comprend une forme courbée ayant une partie en boucle (46),
**caractérisé en ce que** la partie en boucle s'étend parallèlement à un axe longitudinal (52) du dispositif d'accès vasculaire (16), la partie en boucle (46) comprenant un axe central (50) s'étendant à travers la partie en boucle (46) et orienté perpendiculairement à l'axe longitudinal (52) du dispositif d'accès vasculaire (16),
dans lequel la partie en boucle (46) est configurée pour saisir ou piéger une occlusion dans une vasculature et/ou pour faciliter le retrait de l'occlusion.

2. Instrument d'accès vasculaire selon la revendication 1, dans lequel la partie intermédiaire (54) est disposée à proximité de l'extrémité distale (34), où le centre de la partie en boucle (66) est orienté perpendiculairement à la partie intermédiaire (54).

3. Instrument d'accès vasculaire selon la revendication 1, dans lequel une pointe distale (56) de l'extrémité distale (34) est dirigée dans une direction proximale.

4. Dispositif d'accès vasculaire selon la revendication 1, dans lequel la partie en boucle (46) comprend l'une parmi une boucle unique et une pluralité de boucles concentriques.

5. Dispositif d'accès vasculaire selon la revendication 1, dans lequel les boucles de la partie en boucle (46) sont mesurées à partir d'un premier point d'alignement ou de contact (48) se déplaçant de manière distale le long de l'instrument d'accès vasculaire (32) depuis l'extrémité proximale (37) du dispositif d'accès vasculaire et dans lequel la partie en boucle (46) est positionnée en dessous de l'axe longitudinal (52) du dispositif d'accès vasculaire (16).
